# EUROPEAN PATENT SPECIFICATION

(11) **EP 1 971 573 B1**
(45) Date of publication and mention of the grant of the patent: **14.10.2009**
(21) Application number: 06850451.3
(22) Date of filing: 22.12.2006
(51) Int. Cl.: C07D 205/08, C07D 263/24

(54) **PROCESSES FOR PREPARING INTERMEDIATE COMPOUNDS USEFUL FOR THE PREPARATION OF EZETIMIBE**
VERFAHREN ZUR HERSTELLUNG VON ZWISCHENPRODUKTEN FÜR DIE HERSTELLUNG VON EZETIMIBE
PROCÉDÉS DE PRÉPARATION D'INTERMÉDIAIRES UTILES POUR LA PRÉPARATION DE L'ÉZÉTIMIBE

(30) Priority: 22.12.2005 US 752589 P
(43) Date of publication of application: 24.09.2008
(73) Proprietor: Medichem S.A., 08970 Sant Joan Despi (ES)
(72) Inventor: GAVALDA I ESCUDE, Ana, Barcelona (ES); BOSCH I LLADO, Jordi, E-17003 Girona (ES); VIDAL I FERRAN, Anton, E-43340 Montbrió del Camp (ES); GARCIA GARCIA, Eva, E-20500 Mondragón (ES)
(74) Representative: Duncan, Garreth Andrew
(86) International application number: PCT/IB2006/004107
(87) International publication number: WO 2007/119106

(56) References cited:
- WO-A-20/07072088
- US-A- 5 767 115
- US-A- 5 856 473

## Description

### CROSS REFERENCE TO ELATED APPLICATIONS

This application claims priority to United States Provisional Application No. 60/752,589, filed December 22, 2005.

### BACKGROUND OF THE INVENTION

### Field of the Invention

The invention relates, in general, to an improved process for the preparation of the compound (3*R*,4*S*)-4-(4-(benzyloxy)phenyl)-1-(4-fluorophenyl)-3-[3-(4-fluorophenyl)-3-oxopropyl]azetidin-2-one, which is a key intermediate for the synthesis of ezetimibe, as well as the use of this intermediate for the preparation of ezetimibe.

Also disclosed is an improved process for converting compounds of Formula II (below), to compounds of Formula I (below), which are key intermediates for the synthesis of ezetimibe, wherein in Formulas II and I, R represents hydrogen, alkyl, or a hydroxyl protecting group (*e.g*., benzyl group, a substituted benzyl group, or a silyl group). The invention further includes the use of the described process and the use of compounds of Formula I made by the described process for the preparation of ezetimibe.

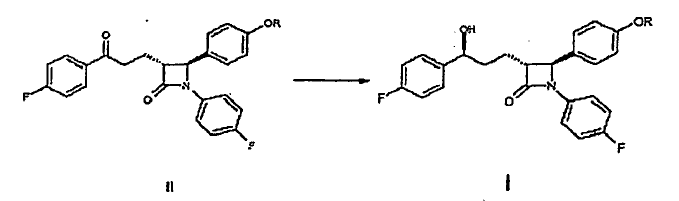

### Discussion of the Related Art

Ezetimibe is a commercially marketed pharmaceutically active substance known to be useful for the treatment of primary hypercholesterolemia, homozygous familial hypercholesterolemia and homozygous familial sitosterolemia. Ezetimibe has an empirical formula of C₂₄H₂₁F₂NO₃ and a molecular weight of 409.4. Ezetimibe is the international common accepted name for (3*R*,4*S*)-1-(4-fluorophenyl)-3-[(3*S*)-3-(4-fluorophenyl)-3-hydroxypropyl]-4-(4-hydroxyphenyl)azetidin-2-one, and its structural formula is: 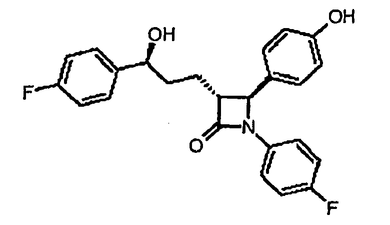 (*i.e*. Compound I above, wherein R = H).

Ezetimibe and its preparation are described in U.S. Patent No. RE 37,721. In this patent, ezetimibe is prepared by the synthetic route shown below:

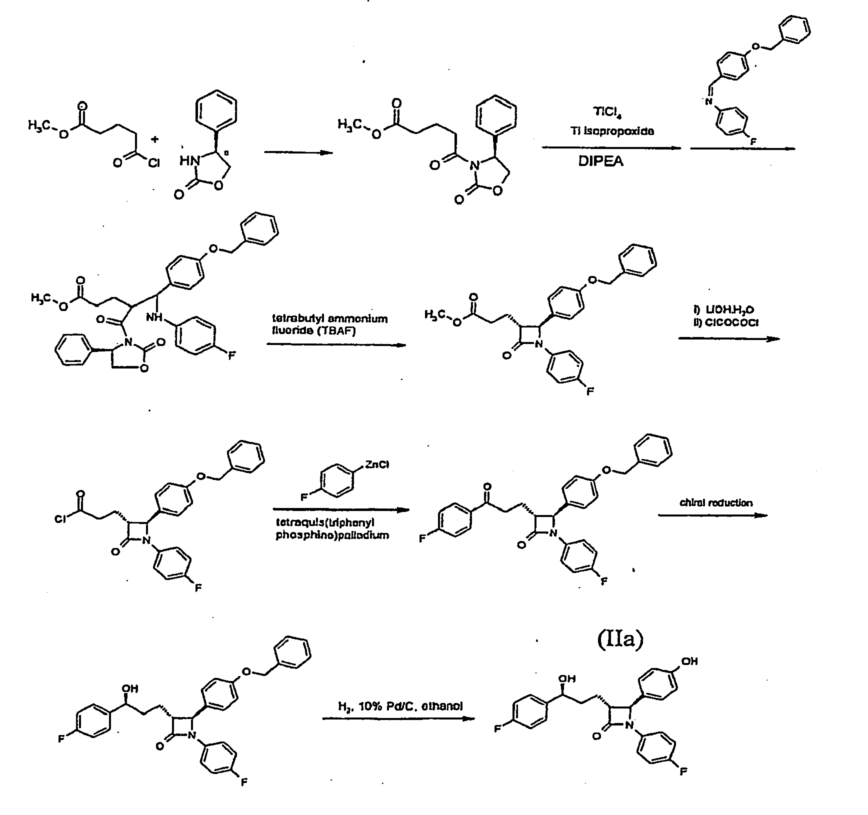

The process described in the U.S. Reissue Patent No. 37,721 and outlined above in Scheme 1 is laborious and involves many steps. As such, there is a need for an improved processes for preparing ezetimibe.

Several processes have been described for preparing ezetimibe in, for example, U.S. Patent Nos. 5,739,321; 5,856,473; and 6,207,822.

U.S. Patent No. 5,739,321 describes a process for preparing ezetimibe by reacting γ-lactam and an imine to give an azetidinone containing a diol group, which is oxidized to the corresponding aldehyde and then condensed with an enolether. The resulting intermediate is then hydrogenated followed by a chiral catalytic reduction and a debenzylation to yield ezetimibe.

U.S. Patent No. 5,856,473 describes preparing ezetimibe by oxidation of a propenyl derivative to obtain the corresponding ketone, which is then reduced and debenzylated.

U.S. Patent No. 6,207,822 describes preparing ezetimibe by reacting *p-*fluorobenzoylbutyric acid with pivaloyl chloride followed by acylation of the obtained product with a chiral auxiliary. Next, reduction of a keto group is performed using a chiral catalyst. The chiral alcohol thus obtained is then reacted with an imine and a silyl protecting agent to give a β-(substituted-amino)amide, which is cyclized and then deprotected to yield ezetimibe.

### SUMMARY OF THE INVENTION

The invention relates, in general, to an improved process for the preparation of the compounds (3*R*,4*S*)-4-(4-(benzyloxy)phenyl)-1-(4-fluorophenyl)-3-[3-(4-fluorophenyl)-3-oxopropyl]azetidin-2-one and (3*R*,4*S*)-1-(4-fluorophenyl)-3-[3-(4-fluorophenyl)-3-oxopropyl]-4-(4-hydroxyphenyl)-azetidin-2-one, which are key intermediates for the synthesis of ezetimibe, as well as the use of these intermediates for the preparation of ezetimibe.

Also disclosed is an improved process for converting compounds of Formula II to compounds of Formula I, which are key intermediates for the synthesis of ezetimibe. The invention further includes the use of the described process and the use of compounds of Formula I made by the described process for the preparation of ezetimibe.

In particular, the invention provide a process for preparing compounds of general Formula II, 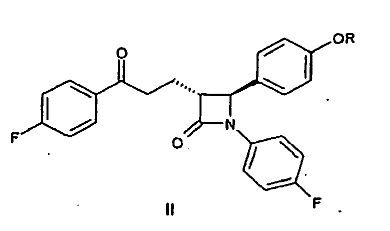 wherein R represents hydrogen, alkyl, or a hydroxyl protecting group (*e.g*., a benzyl group, a substituted benzyl group or a silyl group). In one preferred process, illustrated in Scheme A below, R is a benzyl group. In another preferred process, illustrated in Scheme B below, R is trimethylsilyl (TMS) or hydrogen.

Another aspect of the invention includes a process for preparing the compounds (3*R*,4*S*)-4-(4-(benzyloxy)phenyl)-1-(4-fluorophenyl)-3-[3-(4-fluorophenyl)-3-oxopropyl] azetidin-2-one and (3*R*,4*S*)-1-(4-fluorophenyl)-3-[3-(4-fluorophenyl)-3-oxopropyl]-4-(4-hydroxyphenyl)-azetidin-2-one, as depicted in Formula IIa and IIb (below), which are key intermediate for the synthesis of ezetimibe. 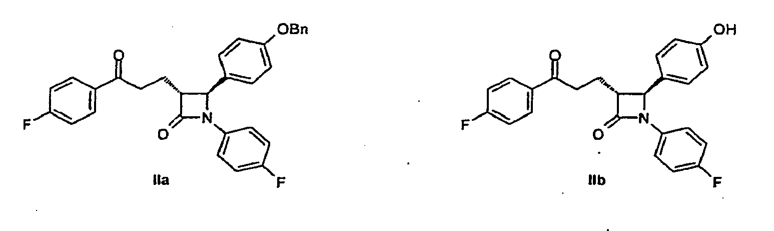 The process includes

### i. reacting the ketone of Formula III (below)

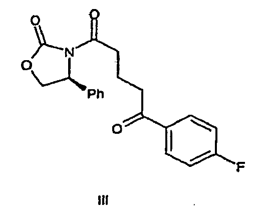

with a diol to obtain the ketal of Formula IV (below), 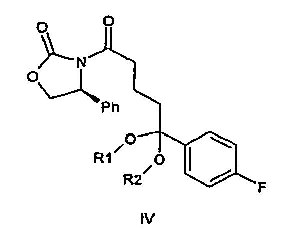 wherein R1 and R2 are together an ethylene or trimethylene diradical, optionally, substituted with a C₁₋₄-alkyl chain;

### ii. condensing the ketal of Formula IV with an imine of Formula V (below)

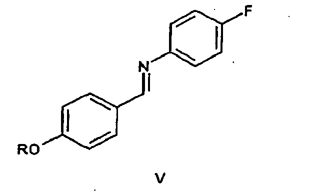

wherein R represents hydrogen, alkyl or a hydroxyl protecting group, to obtain an amide of Formula VI (below), 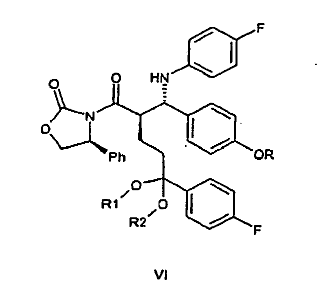 wherein R, R1 and R2 are as defined above;

### iii. cyclizing the amide of step ii to obtain a lactam of formula VII,

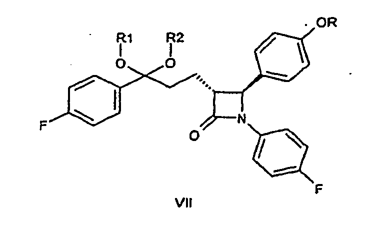

wherein R, R1 and R2 are as defined above; and

### iv. cleaving the ketal function to obtain a compound of Formula II.

In the above described process, a preferred compound of Formula IV is where R1 and R2 are together an ethylene diradical (*i.e*., where the compound of Formula IV is (*S*)-3-{4-[2-(4-fluorophenyl)-[1,3]-dioxolan-2-yl]butyryl}-4-phenyloxazolidin-2-one). (*S*)-3-{4-[2-(4-fluoro phenyl)-[1,3]-dioxolan-2-yl]butyryl}-4-phenyloxazolidin-2-one has not previously been reported in the chemical literature and, as described herein, is useful for the preparation of ezetimibe.

In the above-described process, one preferred compound of Formula V is where R is a benzyl group (*i.e*., where the compound of Formula V is 4-benzyloxybenzylidene-4-fluoroaniline). In another preferred compound of Formula V, R is hydrogen (*i.e.,* where the compound of Formula V is 4-[[(4-fluorophenyl)imino]methyl]phenol).

In the above-described process, one preferred compound of Formula VI is where R1 and R2 are together an ethylene diradical and R is a benzyl group (*i.e*., where the compound of Formula VI is (*S*)-3-{(*R*)-2-[(*S*)-(4-(benzyloxyphenyl))-(4-fluorophenylamino)methyl]-4-[2-(4-fluorophenyl)-[1,3]-dioxolan-2-yl]butyryl}-4-phenyloxazolidin-2-one). (*S*)-3-{(*R*)-2-[(*S*)-(4-(benzyloxyphenyl))-(4-fluorophenylamino)methyl]-4-[2-(4-fluorophenyl)-[1,3]-dioxolan-2-yl]butyryl}-4-phenyloxazolidin-2-one has not previously been reported in the chemical literature and, as described herein, is useful for the preparation of ezetimibe.

In another preferred compound of Formula VI, R1 and R2 are together an ethylene diradical, and R is a hydrogen atom (*i.e*., where the compound of Formula VI is (*S*)-3-{(*R*)-2-[(*S*)-(4-fluorophenylamino)-(4-hydroxyphenyl)methyl]-4-[2-(4-fluorophenyl)-[1,3]-dioxolan-2-yl]butyryl}-4-phenyloxazolidin-2-one). (*S*)-3-{(*R*)-2-[(*S*)-(4-fluorophenylamino)-(4-hydroxyphenyl)methyl]-4-[2-(4-fluorophenyl)-[1,3]-dioxolan-2-yl]butyryl}-4-phenyloxazolidin-2-one has not previously been reported in the chemical literature and, as described herein, is useful for the preparation of ezetimibe.

In a most preferred compound of Formula VI, R1 and R2 are together an ethylene diradical, and R is a trimethylsilyl group (*i.e*., where the compound of Formula VI is (*S*)-3-{(*R*)-2-[(*S*)-(4-fluorophenylamino)-(4-trimethylsilyloxyphenyl)methyl]-4-[2-(4-fluorophenyl)-[1,3]-dioxolan-2-yl]butyryl}-4-phenyloxazolidin-2-one). (*S*)-3-{(*R*)-2-[(*S*)-(4-fluorophenylamino)-(4-trimethylsilyloxyphenyl)methyl]-4-[2-(4-fluorophenyl)-[1,3]-dioxolan-2-yl]butyryl}-4-phenyloxazolidin-2-one has not previously been reported in the chemical literature and, as described herein, is useful for the preparation of ezetimibe.

In the above-described process, one preferred compound of Formula VII is where R1 and R2 are together an ethylene diradical and R is a benzyl group (*i.e*., where the compound of Formula VII is (3*R*,4*S*)-4-(4-(benzyloxyphenyl)-1-(4-fluorophenyl)-3-{2-[2-(4-fluorophenyl)-[1,3]-dioxolan-2-yl]ethyl}azetidin-2-one). (3*R*,4*S*)-4-(4-(benzyloxyphenyl)-1-(4-fluorophenyl)-3-{2-[2-(4-fluorophenyl)-[1,3]-dioxolan-2-yl]ethyl}azetidin-2-one has not previously been reported in the chemical literature and, as described herein, is useful for the preparation of ezetimibe.

In another preferred compound of Formula VII, R1 and R2 are together an ethylene diradical, and R is a trimethylsilyl group *(i.e.,* where the compound of Formula VII is (3*R*,4*S*)-1-(4-fluorophenyl)-3-{2-[2-(4-fluorophenyl)-1,3-dioxolan-2-yl]ethyl}-4-(4-trimethylsilyloxy phenyl)-azetidin-2-one). (3*R*,4*S*)-1-(4-fluorophenyl)-3-{2-[2-(4-fluorophenyl)-1,3-dioxolan-2-yl]ethyl}-4-(4-trimethylsilyloayphenyl)-azetidin-2-one has not previously been reported in the chemical literature and, as described herein, is useful for the preparation of ezetimibe.

A most preferred compound of Formula VII is where R1 and R2 are together an ethylene diradical and R is a hydrogen atom (*i.e*., where the compound of Formula VII is (3*R*,4*S*-1-(4-fluorophenyl)-3-{2-[2-(4-fluorophenyl)-1,3-dioxolan-2-yl]ethyl)-4-(4-hydroxyphenyl)-azetidin-2-one). (3*R*,4*S*)-1-(4-fluorophenyl)-3-{2-[2-(4-fluorophenyl)-1,3-dioxolan-2-yl]ethyl}-4-(4-hydroxyphenyl)-azetidin-2-one has not previously been reported in the chemical literature and, as described herein, is useful for the preparation of ezetimibe.

In the above-described process, a preferred compound of Formula II is where R is a benzyl group (*i.e*., where the compound of Formula II is (3*R*,4*S*)-4-(4-(benzyloxy)phenyl)-1-(4-fluorophenyl)-3-[3-(4-fluorophenyl)-3-oxopropyl]azetidin-2-one). Most preferably, R is hydrogen (*i.e*., where the compound of Formula II is (3*R*,4*S*)-1-(4-fluorophenyl)-3-[3-(4-fluorophenyl)-3-oxopropyl] -4-(4-hydroxyphenyl)-azetidin-2-one).

Another aspect of the invention includes a process for preparing ezetimibe from the compounds of Formula IIa and IIb.

Another aspect of the invention includes a process for preparing ezetimibe that includes the above-described process for preparing the compounds of Formula IIa and IIb.

Additional advantages and features of the invention will become apparent from the detailed description which follows.

### DETAILED DESCRIPTION OF THE PREFERRED EMBODIMENTS

Reference will now be made in detail to the preferred embodiments of the invention.

One aspect of the invention includes a process for preparing compounds of general Formula II, 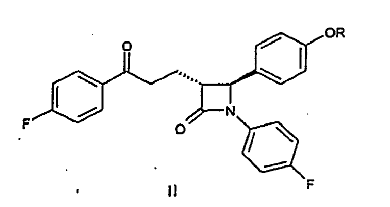 wherein R represents hydrogen, alkyl or a hydroxyl protecting group. In one embodiment, R is preferably a benzyl group. In another embodiment, R is preferably trimethylsilyl or hydrogen.

Another aspect of the invention includes a process for preparing the compound 3*R*,4*S*)-4-(4-(benzyloxy)phenyl)-1-(4-fluorophenyl)-3-[3-(4-fluorophenyl)-3-oxopropyl] azetidin-2-one, as depicted in Formula IIa (below), which is a key intermediate for the synthesis of ezetimibe.

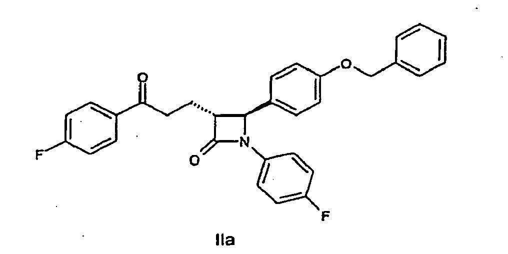

Another aspect of the invention includes a process for preparing the compound (3*R*,4*S*)-1-(4-fluorophenyl)-3-[3-(4-fluorophenyl)-3-oxopropyl]-4-(4-hydroxyphenyl)-azetidin-2-one, as depicted in Formula IIb (below), which is a key intermediate for the synthesis of ezetimibe. 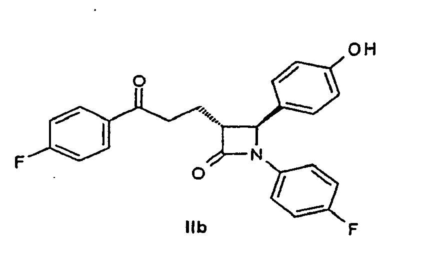 The process includes

### i. reacting the ketone of Formula III (below)

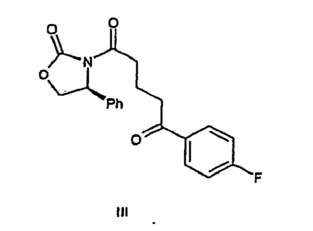

with a diol to obtain the ketal of Formula IV (below), 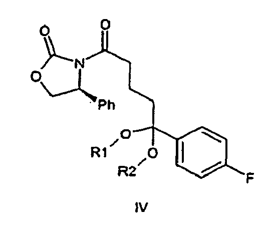 wherein R1 and R2 are together an ethylene or trimethylene diradical, optionally, substituted with a C₁₋₄-alkyl chain;

### ii. condensing the ketal of Formula IV with an imine of Formula V (below)

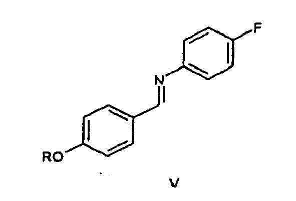

wherein R represents hydrogen, alkyl, or a hydroxyl protecting group, to obtain an amide of Formula VI (below), 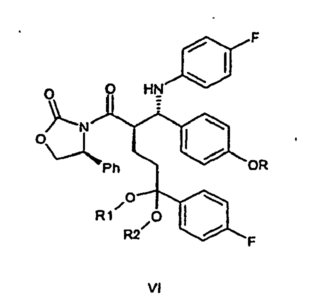 wherein R, R1 and R2 are as defined above;

### iii. cyclizing the amide of step iii to obtain a lactam of formula VII,

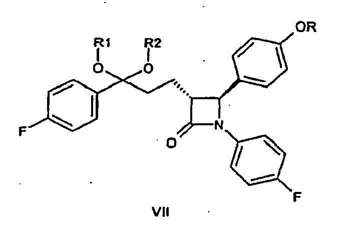

wherein R, R1 and R2 are as defined above; and

### iv. cleaving the ketal function to obtain a compound of Formula II.

Thus compounds of Formula II, for example, Formula IIa and IIb, can be prepared as follows (as will be discussed below, compounds of Formula II can be further converted into ezetimibe, for example, by chiral reduction (step v) and deprotection/benzydrolysis (step vi), if necessary) as illustrated in Schemes A and B, below:

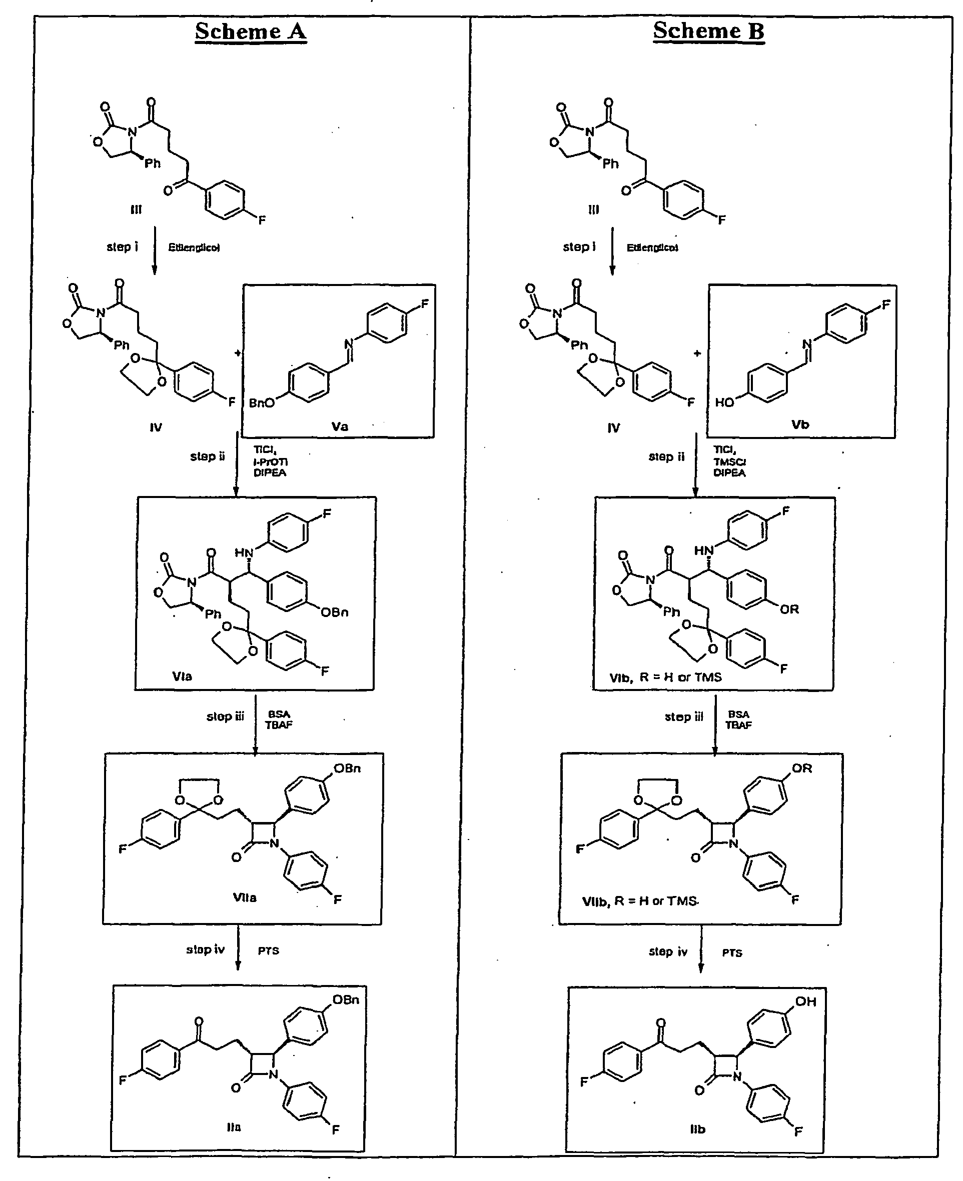

Another aspect of the invention includes using in the above-described process a compound of Formula IV where R1 and R2 are together an ethylene diradical (*i.e*., where the compound of Formula IV is (*S*)-3-{4-[2-(4-fluorophenyl)-[1,3]-dioxolan-2-yl]butyryl}-4-phenyloxazolidin-2-one).

Another aspect of the invention includes the use of (*S*')-3-{4-[2-(4-fluorophenyl)-[1,3]-dioxolan-2-yl]butyryl}-4-phenyloxazolidin-2-one in the preparation of ezetimibe.

Another aspect of the invention includes using in the above-described process a compound of Formula V wherein R is a benzyl group (*i.e*., where the compound of Formula V is 4-benzyloxybenzylidene-4-fluoroaniline). Alternatively, R may be a hydrogen atom *(i.e.,* where the compound of Formula V is 4-[[(4-fluorophenyl)imino]methyl]phenol).

Another aspect of the invention includes using in the above-described process a compound of Formula VI wherein R1 and R2 are together an ethylene diradical and R is a hydrogen or a protecting group. Preferred protecting groups are trimethylsilyl and benzyl groups, with trimethylsilyl being most preferred (*i.e*., where the compound of Formula VI is (*S*)-3- {(*R*)-2-[(*S*)-(4-fluorophenylamino)-(4-trimethylsilyloxyphenyl)methyl]-4-[2-(4-fluorophenyl)-[ 1,3]-dioxolan-2-yl]butyryl}-4-phenyloxazolidin-2-one).

Another aspect of the invention includes the use of (*S*)-3-{(*R*)-2-[(*S*)-(4-(benzyloxy phenyl))-(4-fluorophenylamino)methyl]-4-[2-(4-fluorophenyl)-[1,3]-dioxolan-2-yl]butyryl}-4-phenyloxazolidin-2-one, (*S*)-3-{(*R*)-2-[(*S*)-(4-fluorophenylamino)-(4-trimethylsilyloxy phenyl)methyl]-4-[2-(4-fluorophenyl)-[1,3]-dioxolan-2-yl]butyryl} -4-phenyloxazolidin-2-one or (*S*)-3-{(*R*)-2-[(*S*)-(4-fluorophenylamino)-(4-hydroxyphenyl)methyl]-4-[2-(4-fluorophenyl)-[1,3]-dioxolan-2-yl]butyryl}-4-phenyloxazolidin-2-one in the preparation of ezetimibe.

Another aspect of the invention includes using in the above-described process a compound of Formula VII wherein R1 and R2 are together an ethylene diradical and R is hydrogen or a protecting group. Preferred protecting groups are trimethylsilyl and benzyl groups, with trimethylsilyl being most preferred (*i.e*., where the compound of Formula VII is (3*R*,4*S*)-1-(4-fluorophenyl)-3-{2-[2-(4-fluorophenyl)-1,3-dioxolan-2-yl]ethyl}-4-(4-hydroxyphenyl)-azetidin-2-one.

Another aspect of the invention includes the use of (3*R*,4*S*)-4-(4-(benzyloxyphenyl)-1-(4-fluorophenyl)-3-{2-[2-(4-fluorophenyl)-[1,3]-dioxolan-2-yl]ethyl azetidin-2-one, (3*R*,4*S*)-1-(4-fluorophenyl)-3-{2-[2-(4-fluorophenyl)- [1,3]-dioxolan-2-yl] ethyl }-4-(4-trimethylsilyloxyphenyl)-azetidin-2-one or (3*R*,4*S*)-1-(4-fluorophenyl)-3-{2-[2-(4-fluorophenyl)-[1 ,3]-dioxolan-2-yl]ethyl} -4-(4-hydroxyphenyl)-azetidin-2-one in the preparation of ezetimibe.

Another aspect of the invention includes using in the above-described process a compound of Formula II wherein R is a benzyl group (*i.e*., where the compound of Formula II is (3*R*,4*S*')-4-(4-(benzyloxy)phenyl)-1-(4-fluorophenyl)-3-[3-(4-fluorophenyl)-3-oxopropyl]azetidin-2-one) or hydrogen *(i.e.,* where the compound of Formula II is (3*R*,4*S*)-1-(4-fluorophenyl)-3-[3-(4-fluorophenyl)-3-oxopropyl]-4-(4-hydroxyphenyl)-azetidin-2-one).

Another aspect of the invention includes a process for preparing ezetimibe from the compounds of Formula IIa and IIb.

Another aspect of the invention includes a process for preparing ezetimibe that includes the above-described process for preparing the compounds of Formula IIa and IIb.

Another aspect of the invention includes using in the above-described process a compound of Formula III that is commercially available or that have been prepared by methods known in the art.

Another aspect of the invention includes using in the above-described process a compound of Formula V, where R is a benzyl group or hydrogen, that is commercially available or that have been prepared by methods known in the art.

Another aspect of the invention includes in step i of the above-described process reacting a compound of Formula III with a diol using an acid as a catalyst, optionally using a solvent, and at a temperature between approximately 10° C and approximately 150°C and then isolating a compound of Formula IV by conventional extraction methods. In this aspect of the invention, the diol is preferably a glycol and more preferably ethylene glycol, the acid catalyst is preferably *p-*toluenesulfonic acid or chlorotrimethylsilane, and the optional solvents are preferably toluene, dichloromethane or mixtures thereof.

Another aspect of the invention includes in step ii of the above-described process that the ketal of Formula IV is added to a solution of titanium isopropoxide and a Lewis acid in an anhydrous solvent at a temperature of approximately -10° C to approximately 50° C, and preferably approximately 0°C. Then, a tertiary amine base is added at a temperature of approximately -10° C to approximately 50°C, and preferably approximately 0°C. Imine of Formula V is then added at a temperature of approximately 0° C to approximately -50° C, and preferably approximately -15°C and stirred for approximately 2 hours to approximately 20 hours, and preferably approximately 17 hours. The reaction is then quenched, and the resulting product of Formula VI, wherein R1 and R2 are together an ethylene diradical and R is a benzyl group, is isolated by conventional extraction methods, and optionally purified by crystallization with a solvent such as ethanol or acetonitrile.

Another aspect of the invention includes in step ii of the above-described process suspending the ketal of Formula IV and the imine of Formula V, wherein R is hydrogen, in an anhydrous solvent at a temperature of approximately -20°C to approximately 20°C, preferably approximately -10°C. Then, a tertiary amine base is added at a temperature of approximately -20°C to approximately 20°C, preferably approximately -10°C. Chlorotrimethylsilane is added at a temperature of approximately -20°C to approximately 20° C, preferably approximately -10° C, and stirred for approximately 30 minutes to approximately 2 hours, preferably approximately 1 hour. Then, titanium tetrachloride is added a at a temperature of approximately -50°C to approximately 0°C, preferably approximately -15°C, and stirred for approximately 2 hours to approximately 20 hours, preferably approximately 17 hours. The reaction is then quenched, and the resulting product of Formula VI, wherein R1 and R2 are together an ethylene diradical and R is hydrogen or a trimethylsilyl group, is isolated by conventional extraction methods. The obtained product can be optionally purified by crystallization with a solvent such as ethanol or acetonitrile.

In the two foregoing aspects of the invention, the Lewis acid is preferably a titanium or zirconium derivative, and more preferably a titanium derivative with the general formula (i-PrO)_{y} TiClₓ, where x + y = 4. Preferably the anhydrous solvent is dichloromethane. Preferably the tertiary amine base is diisopropylethylamine. Preferably approximately 1 to approximately 3 equivalents of the imine of Formula V are used, and more preferably approximately 2 equivalents. Additionally in the two foregoing aspects of the invention, the resulting product can be optionally purified by crystallization with a solvent such as an alcohol, preferably ethanol.

Another aspect of the invention includes in step iii of the above-described process that the compound of Formula VI is treated with a mild silylating agent at approximately 0° C to approximately 100°C, preferably at approximately 50° C, using a solvent or a mixture of solvents, for approximately 10 minutes to approximately 60 minutes, preferably approximately 30 minutes. The mixture is then treated with a fluoride anion source at approximately 0°C to approximately 100° C, preferably at approximately 50°C, and stirred for approximately 0.5 hours to approximately 4 hours, preferably for approximately 3 hours before the resulting product is isolated by conventional extraction methods. In this aspect of the invention, the silylating agent is preferably N,O-bis(trimethylsilyl)acetamide, the solvent is preferably toluene, and the fluoride anion source is preferably tetrabutylammonium fluoride (TBAF).

Another aspect of the invention includes in step iv of the above-described process that a solution of the azetidinone of Formula VII in an inert solvent and a deprotecting agent is heated at approximately 40° C to approximately 100° C, and preferably at approximately 56°C, with an acid catalyst for approximately 4 hours to approximately 8 hours, and preferably approximately 6 hours before the resulting product is isolated by conventional extraction methods. In this aspect of the invention, acetone is preferably wet acetone and is used as both the deprotecting agent and solvent, and *p-*toluenesulfonic acid is preferably used as the acid catalyst.

Another aspect of the invention includes certain preferred reactants and conditions in the above-described process. In step i of this preferred aspect of the above-described process, the compound of Formula III is reacted with ethylene glycol using an acid as catalyst, preferably *p-*toluenesulfonic acid or chlorotrimethylsilane, optionally using a solvent, preferably toluene or dichloromethane and at a temperature between approximately 10°C, and approximately 150°C. The resulting compound of Formula IV where R1 and R2 are together an ethylene diradical is isolated by conventional extraction methods.

In step ii of one preferred aspect of the above-described process, the ketal of Formula IV is condensed with the imine of Formula V where R is a benzyl group. In particular, one equivalent of the ketal is added to a solution of titanium isopropoxide and titanium tetrachloride, in an anhydrous solvent such as dichloromethane at a temperature of approximately -10° C to approximately 50°C, preferably approximately 0°C. Then, diisopropylethylamine is added at a temperature of approximately -10°C to approximately 50°C, preferably approximately 0° C. Imine of Formula V, preferably approximately 1 to approximately 3 equivalents, is then added at a temperature of approximately 0° C to approximately -50°C, preferably approximately -15° C, and stirred for approximately 2 hours to approximately 20 hours, preferably approximately 17 hours. The reaction is then quenched, for example by treating with an acid such as acetic acid or sulfuric acid, and the resulting product of Formula VI, where R1 and R2 are together an ethylene diradical and R is a benzyl group, is isolated by conventional extraction methods. In this aspect of the invention, the product can be optionally purified by crystallization with a solvent such as ethanol.

In step ii of another preferred aspect of the above-described process, the ketal of Formula IV is condensed with the imine of Formula V where R is hydrogen. In particular, 1 equivalent of the ketal and approximately 1 to 3 equivalents of the imine of Formula V, preferably 2 equivalents, are suspended in an anhydrous solvent such as dichloromethane at a temperature of approximately -20° C to approximately 20° C, preferably approximately -10° C. Then, approximately 2 to 4 equivalents of diisopropylethylamine, preferably 3.5 equivalents, are added at a temperature of approximately -20° C to approximately 20° C, preferably approximately -10° C. Chlorotrimethylsilane, approximately 1 to 3 equivalents, preferably 2.2 equivalents, is added at a temperature of approximately -20° C to approximately 20° C, preferably approximately -10° C, and stirred for approximately 30 minutes to approximately 2 hours, preferably approximately 1 hour. Then, titanium tetrachloride, approximately 1 to 3 equivalents, preferably 1.1 equivalents, is added at a temperature of approximately -50°C to approximately 0°C, preferably approximately -15° C, and stirred for approximately 2 hours to approximately 20 hours, preferably approximately 17 hours. The reaction is then quenched, for example by treating with an acid such as acetic acid or sulfuric acid, and the resulting product of Formula VI, where R1 and R2 are together an ethylene diradical and R is hydrogen or a trimethylsilyl group, is isolated by conventional extraction methods. In this aspect of the invention, the product can be optionally purified by crystallization with a solvent such as ethanol.

In step iii of this preferred aspect of the above-described process, the compound of Formula VI is treated with N,O-bis(trimethylsilyl)acetamide at approximately 0° C to approximately 100° C, preferably at approximately 50° C, using toluene for approximately 10 minutes to approximately 60 minutes, preferably for approximately 30 minutes. Then the mixture is treated with tetrabutylammonium fluoride (TBAF) at about approximately 0° C to approximately 100° C, preferably at approximately 50° C, and stirred for approximately 0.5 hours to approximately 4 hours, preferably for approximately 3 hours. The resulting product of Formula VII, where R1 and R2 are together an ethylene diradical and R is hydrogen or a hydroxyl protecting group, such as a trimethylsilyl group or a benzyl group, is isolated by conventional extraction methods.

In step iv of this preferred aspect of the above-described process, the azetidinone of Formula VII, where R1 and R2 are together an ethylene diradical and R is hydrogen or a hydroxyl protecting group, such us a trimethylsilyl group or a benzyl group, is heated in acetone at approximately 40° C to approximately 100° C, preferably at approximately 56° C, with *p*-toluenesulfonic acid for approximately 4 hours to approximately 8 hours, preferably for approximately 6 hours. The resulting product of Formula II is isolated by conventional extraction methods. An alternative to *p-*toluenesulfonic acid is the use of sulfuric acid.

As noted above, another aspect of the invention includes, in the above described processes, additional processing steps, including a step v that includes chiral reduction of the intermediate of compounds of Formula II (*e.g*., compounds of Formula IIa and IIb) and, if necessary, a step vi that includes deprotection/benzydrolysis, to give ezetimibe, as illustrated in the following continuations of Schemes A and B:

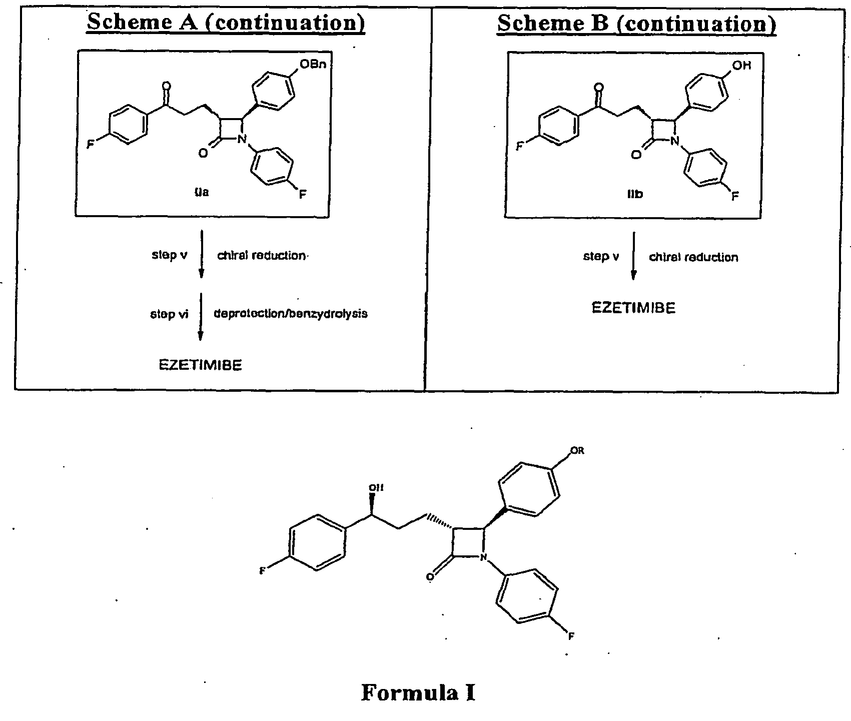

In Scheme A, the order of steps v and vi is not critical to the preparation of the azetidinone compound.

Another aspect of the invention includes in the above-described chiral reduction of step v performing such chiral reduction using homogeneous asymmetric hydrogenation.

Another aspect of the invention includes in the above-described chiral reduction of step v performing such chiral reduction using homogeneous asymmetric reduction where the catalyst used for such asymmetric reduction is a ruthenium- or rhodium-based catalyst coupled with a chiral ligand.

The compounds of Formula II (*e.g.,* (3*R*,4*S*)-4-(4-(benzyloxy)phenyl)-1-(4-fluorophenyl)-3-[3-(4-fluorophenyl)-3-oxopropyl]azetidin-2-one) can be converted to compounds of Formula I (*e.g*., (3*R*,4*S*)-4-(4-(benzyloxy)phenyl)-1-(4-fluorophenyl)-3-[(3*S*)-3-(4-fluorophenyl)-3-hydroxypropyl]azetidin-2-one) via asymmetric reduction of the phenone-type ketone of the compounds of Formula II.

Compounds of Formula I can be prepared via the above described asymmetric reduction process from the compounds (3*R*,4*S*)-4-(4-(benzyloxy)phenyl)-1-(4-fluorophenyl)-3-[3-(4-fluorophenyl)-3-oxopropyl] azetidin-2-one, as depicted in Formula IIa, (3*R*,4*S*)- 1-(4-fluorophenyl)-3-[3-(4-fluorophenyl)-3-oxopropyl]-4-(4-(hydroxyphenyl) azetidin-2-one and (3*R*,4*S*)-4-(4-(trimethylsilyloxy)phenyl)-1-(4-fluorophenyl)-3-[3-(4-fluorophenyl)-3-oxopropyl] azetidin-2-one.

Another aspect of the invention includes the use of compounds of Formula I prepared from compounds of Formula II by the above-described asymmetric reduction process to prepare ezetimibe.

The various embodiments of the invention having thus been generally described, several examples will hereafter be discussed to illustrate the inventive aspects more fully.

### Specific Examples

The following examples are for illustrative purposes only and are not intended, nor should they be interpreted to, limit the scope of the invention.

### General Experimental Conditions:

### HPLC Chiral Method

The chromatographic separation was carried out in a Daicel CHIRALCEL OD-H, 5 µm, 4.6 x 150 mm column at room temperature (20-25° C).

The mobile phase was prepared by mixing 950 mL of hexane with 50 mL of ethanol. The mobile phase was mixed and filtered through 0.22 µm nylon membrane under vacuum.

The chromatograph was equipped with a 232 nm detector and the flow rate was 1 mL per minute. Test samples (10 µl) were prepared by dissolving a sufficient quantity of sample in order to obtain a 0.5 mg per mL concentration in the mobile phase. Following sample injection, the chromatogram was run for at least 60 minutes.

### EXAMPLE 1: Preparation of (S)-3-{4-[2-(4-fluorophenyl)-[1,3]-dioxolan-2-yl]butyryl}-4-phenyloxazolidin-2-one (Compound IV)

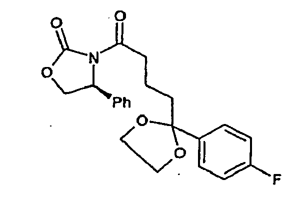

**Method A:** In a 250 mL flask, chlorotrimethylsilane (7.2 mL, 56.3 mmol) was added to a suspension of (*S*)-3-[5-(4-fluorophenyl)-1,5-dioxopentyl]-4-phenyloxazolidin-2-one (5.00 g, 14.1 mmol) in ethylene glycol (70.00 g, 1.13 mol) at 20-25° C. The reaction was stirred at this temperature for 20 hours. Then, a 5% aqueous sodium hydrogencarbonate solution (60 mL) and toluene (40 mL) were added. The resulting biphasic system was heated at 50°C and stirred for 30 minutes. The phases were then separated at 50° C. The organic phase was further washed at 50°C with 5% aqueous sodium hydrogencarbonate (30 mL) and water (30 mL). The organic phase was then dried over MgSO₄. After evaporation of the solvent under vacuum, (*S*)-3-{4-[2-(4-fluorophenyl)-[1,3]-dioxolan-2-yl]butyryl}-4-phenyloxazolidin-2-one was obtained as a white solid (5.59 g; Yield: 95%; 95% purity; 13.4 mmol). The product was used in the following steps (below) without further purification.

**Method B:** In a 50 mL flask, to a solution of (*S*)-3-[5-(4-fluorophenyl)-1,5-dioxopentyl]-4-phenyloxazolidin-2-one (2.00 g, 5.62 mmol) in 14.1 mL of toluene was added ethylene glycol (1.10 g, 17.66 mmol) and *p-*toluenesulfonic acid (40 mg, 0.19 mmol). The resulting solution was heated at reflux temperature using a Dean-Stark trap for 16 hours. The reaction mixture was then cooled to room temperature, washed with saturated sodium hydrogenocarbonate solution, brine and then dried over Na₂SO₄. After evaporation of the solvent under vacuum, (*S*)-3-{4-[2-(4-fluorophenyl)-[1,3]-dioxolan-2-yl]butyryl}-4-phenyloxazolidin-2-one was obtained as a white solid.

**Method C:** To a solution of (*S*)-3-[5-(4-fluorophenyl)-1,5-dioxopentyl]-4-phenyloxazolidin-2-one (20.0 g, 56.3 mmol) in toluene (268 ml) at 20-25° C was added ethylene glycol (47.1 mL, 0.84 mol) and chlorotrimethylsilane (21.6 mL, 0.17 mol), and the mixture was stirred at this temperature for 48 hours. Then, agitation was stopped, and the bottom layer was discarded. The organic layer was washed three times with a 5% aqueous sodium hydrogencarbonate solution (50 mL) and water (50 mL). Next, the organic layer was concentrated at vacuum yielding (*S*)-3-{4-[2-(4-fluorophenyl)-[1,3]-dioxolan-2-yl]butyryl}-4-phenyloxazolidin-2-one (22.0 g; Yield: 89%; 91% purity; 50.1 mmol). The product was used in the following steps (below) without further purification.

*Analysis:* **¹H-NMR (400 MHz, CDCl₃) (**δ**, ppm)**: 7.26-7.38 (m, 7H), 6.97 (t, *J* = 8.3 Hz, 2H), 5.39 (dd, *J*= 3.4, 8.6 Hz, 1H), 4.66 (t, *J*= 8.9 Hz, 1H), 4.25 (dd, *J*= 3.7, 8.6 Hz, 1H), 3.92-4.01, 3.68-3.76 (2 x m, 4H), 2.94 (t, *J*= 7.2 Hz, 2H), 1.82-1.89 (m, 2H), 1.63-1.60 (m, 2H); **¹³C-NMR (125 MHz, CDCl₃) (**δ**, ppm)**: 172.4, 163.6, 161.2, 139.1, 138.1, 129.1, 128.6, 127.5, 127.4, 125.9, 125.8, 114.9, 114.7, 109.8, 69.9, 64.5, 57:5, 39.4, 35.2, 18.2; **MS (ESI +)**: m/z (%) = 422 ([M+Na]⁺, 100).

### EXAMPLE 2: Preparation (S)-3-{(R)-2-[(S)-(4-(benzyloxyphenyl))-(4-fluoro phenylamino)methyl]-4-[2-(4-fluorophenyl)-[1,3]-dioxolan-2-yl]butyryl}-4-phenyloxazolidin-2-one (Compound VIa)

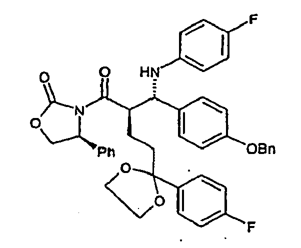

In a 100 mL flask, titanium tetraisopropoxide (0.95 mL, 98%, 3.1 mmol) was added drop wise to a 1M solution of TiCl₄ (9.6 mL, 9.6 mmol), which was cooled previously at 0°C under N₂. The mixture was stirred for 20 minutes. Next, a solution of (*S*)-3-{4-[2-(4-fluorophenyl)-[1,3]-dioxolan-2-yl]butyryl}-4-phenyloxazolidin-2-one (4.18 g, 95% purity, 10.0 mmol) in dichloromethane (13 mL) was added, and the reaction mixture was stirred for 10 minutes at 0° C. Diisopropylethylamine (3.9 mL, 22.2 mmol) was then added, and the mixture was further stirred for 1 hour at 0° C. The solution was then cooled to -15°C, and 4-benzyloxybenzylidine(4-fluoro)aniline (5.87 g, 19.2 mmol) was added as a suspension in 25 mL dichloromethane. The suspension was stirred at -15° C for 17 hours. The reaction was quenched by adding 7.7 mL of acetic acid drop wise at -15° C. Then, the reaction mixture was allowed to warm to 0°C, and sulfuric acid 1M (38 mL) was added. The reaction mixture was stirred at 0° C for 30 minutes, and then allowed to warm to room temperature. The phases were then separated, and the aqueous layer was extracted with dichloromethane (10 mL). The combined organic solutions were washed with a 20% NaHSO₃ solution (10 mL), dried over magnesium sulfate and concentrated under vacuum. Next, 25 mL of ethanol were added to the reaction mixture and the resulting suspension was heated under reflux for 1hour. The mixture was cooled to 5° C and filtered while cold. The solid was washed with cold ethanol (2 x 6 mL) and dried to yield (*S*)-3-{(*R*)-2-[(*S*)-(4-(benzyloxyphenyl))-(4-fluorophenylamino)methyl]-4-[2-(4-fluorophenyl)-[1,3]-dioxolan-2-yl]butyryl}-4-phenyloxazolidin-2-one (4.07 g; Yield: 49%; 86% purity; 4.9 mmol). The solid was used in Example 5 without any further purification.

*Analysis:* **¹H-NMR (400 MHz, CDCl₃) (**δ**, ppm)**: 7.44-7.25 (m, 7H), 7.16-7.02 (m, 7H), 6.94 (t, *J=* 8.6 Hz, 2H), 6.83 (d, *J*= 8.7, 2H), 6.72 (t, *J*= 8.8, 2H), 6.36 (dd, *J*= 9.0, 4.4 Hz, 2H), 5.43 (dd, *J*= 8.4, 2.8 Hz, 1H), 45.01-4.98 (m, 1H), 5.01 (s, 2H), 4.65 (t, *J =* 8.6 Hz, 1H), 4.55-4.48 (m, 1H), 4.28 (t, *J*= 9.6 Hz, 1H), 4.20 (dd, *J* = 8.8, 2.8 Hz, 1H), 3.94-3.87 (m, 2H), 3.75-3.69 (m, 1H), 3.67-3.61 (m, 1H); 1.87-1.70 (m, 2H), 1.36-1.27 (m, 1H); **¹³C-NMR (125 MHz, CDCl₃) (**δ**, ppm):** 175.1, 163.8, 161.3, 161.2, 158.1, 156.9, 154.7, 154.4, 142.9, 142.8, 138.3, 137.8, 136.9, 133.0, 128.9, 128.6, 128.2, 128.0, 127.9, 127.5, 127.4, 127.3, 125.2, 115.5, 115.3, 114.9, 114.8, 109.7, 69.9, 64.6, 64.2, 61.3, 58.0, 47.7, 37.4, 24.7; **MS (ESI +)**: m/z (%) = 743.3 ([M+K]⁺, 10), 727.3 ([M+Na]⁺, 100), 705.3 ([M+H]⁺, 47).

### EXAMPLE 3: Preparation (S)-3-{(R)-2-[(S)-(4-(benzyloxyphenyl))-(4-fluoro phenylamino)methyl]-4-[2-(4-fluorophenyl)-[1,3]-dioxolan-2-yl]butyryl}-4-phenyloxazolidin-2-one (Compound VIa)

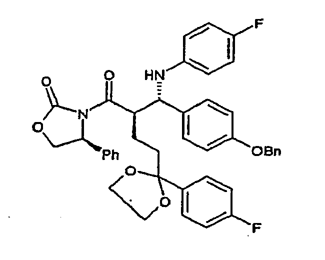

In a 2.0 L flask, titanium tetraisopropoxide (9.31 g, 32.8 mmol) was added dropwise to a 1M solution of TiCl₄ (136.5 g, 0.10 mol), which was cooled previously at 0°C under N₂. The mixture was stirred during 30 min. Next, a solution of (*S*)-3-{4-[2-(4-fluorophenyl)-[1,3]-dioxolan-2-yl]butyryl}-4-phenyloxazolidin-2-one (52.4 g, 0.13 mol) in dichloromethane (476 mL) was added, and the reaction mixture was stirred for 10 min at 0° C. Diisopropylethylamine (36.8 g, 0.28 mol) was then added, and the mixture was further stirred for 1h at 0° C. The solution was then cooled to -15°C, and 4-benzyloxybenzylidine(4-fluoro)aniline (73.3 g, 0.24 mol) was added. The suspension was stirred at -15° C for 4h. The reaction was quenched by adding dropwise at -15°C acetic acid (45.4 mL). Then the reaction mixture was allowed to warm at 0° C, and sulfuric acid 1M (47.6 mL) was added. The reaction mixture was stirred at 0° C for 30 min and then allowed to warm at room temperature. Dichloromethane (300 mL) was added, the phases were separated, and the aqueous layer was extracted with dichloromethane (300 mL). The combined organic solutions were washed with aqueous sodium hydrogencarbonate (300 mL), dried over sodium sulfate and concentrated under vacuum. Ethanol (350 mL) was added to the reaction mixture, and the resulting suspension was heated under reflux for 1h. The mixture was cooled to 5°C and filtered while cold. The solid was washed with cold ethanol (3x10 mL) yielding (*S*)-3-{(*R*)-2-[(*S*)-(4-(beinzyloxyphenyl))-(4-fluorophenylamino)methyl]-4-[2-(4-fluorophenyl)-[1,3]-dioxolan-2-yl]butyryl}-4-phenyloxazolidin-2-one as a solid (54.2 g; Yield: 58%; 92% purity). The purity was increased to 97% with one recrystallization with acetonitile.

### EXAMPLE 4: Preparation of (S)-3-{(R)-2-[(S)-(4-fluorophenylamino)-(4-trimethylsilyloxyphenyl)methyl]-4-[2-(4-fluorophenyl)-[1,3]-dioxolan-2-yl]butyryl}-4-phenyloxazolidin-2-one (Compound VIb)

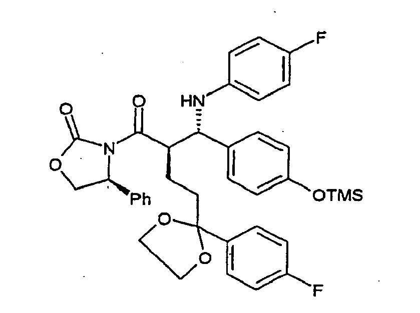

In a 500 mL flask, a mixture of 4-[[(4-fluorophenyl)imino]methyl]phenol (12.04 g (56.18 mmol)) and (*S*)-3-{4-[2-(4-fluorophenyl)-[1,3]-dioxolan-2-yl]butyryl}-4-phenyloxazolidin-2-one (11.22 g, 28.09 mmol) in 40 mL of dichloromethane anhydrous was cooled to -10° C. Then, diisopropylethylamine (17.1 mL, 98.32 mmol) was added dropwise at -10° C. To the resulting reaction mixture, chlorotrimethylsilane (8.1 mL, 61.80 mmol) was added over 30 minutes, and the reaction mixture was stirred at -10°C for one hour. Then, titanium tetrachloride 1M solution (30.1 mL, 30.90 mmol) was added dropwise at -15°C, and the reaction mixture was stirred at -15°C overnight. The reaction was quenched by adding dropwise at -15°C acetic acid (8 mL). Then, the reaction mixture was allowed to warm at 0°C during two hours, and 140 mL of tartaric acid 7% was added in 30 minutes and then allowed to warm to room temperature for two hours. Sodium hydrogen sulfite 20% (50 mL) was added, and the mixture was stirred for two more hours. The mixture was decanted, and the aqueous phase was washed with dichloromethane. The organic phases were combined and washed with water (120 mL) and concentrated under vacuum to 90 mL volume. Then, *N,O-*bis(trimethylsilyl)acetamide (8.4 mL, 34.3 mmol) was added, and the mixture was heated at reflux temperature for 30 minutes. After cooling, the reaction mixture solvent was evaporated under vacuum. Ethanol was added (70 mL) to the reaction mixture, and the resulting suspension was heated under reflux for 1 hour. The mixture was cooled down to 5°C and filtered while cold yielding (*S*')-3-{(*R*)-2-[(*S*)-(4-fluorophenylamino)-(4-trimethylsilyloxyphenyl)methyl]-4-[2-(4-fluorophenyl)-[ 1,3]-dioxolan-2-yl]butyryl}-4-phenyloxazolidin-2-one as a solid (7.87 g; Yield: 41%). The purity was increased with one recrystallization with acetonitile.

*Analysis:* **¹H-NMR (400 MHz, CDCl₃) (**δ**, ppm)**: 7.31-7.24 (m, 2H), 7.18-7.02 (m, 7H), 6.94 (t, *J*= 8.8 Hz, 2H), 6.76-6.67 (in, 4H), 6.37 (dd, *J*= 9.2, 4.4 Hz, 2H), 5.44 (dd, *J* = 8.0, 2.4 Hz, 1H), 4.94 (d, *J* = 10.4 Hz, 1H), 4.66 (t, *J* = 8.4 Hz, 1H), 4.55-4.45 (m, 1H), 4.25 (t, *J*= 10 Hz, 1H), 4.21 (dd, *J*= 8.8, 2.8 Hz, 1H), 3.94-3.86 (m, 2H), 3.75-3.69 (m, 1H), 3.67-3.60 (m, 1H), 1.87-1.68 (m, 3H), 1.33-1.23 (m, 1H), 0.24 (s, 9H); **¹³C-NMR (125 MHz, CDCl₃) (**δ**, ppm)**: 175.1, 163.6, 161.2, 157.0, 154.7, 154.5, 142.9, 142.8, 138.4, 137.9, 137.8, 133.5, 128.9, 128.2, 128.0, 127.5, 127.4, 125.2, 120.0, 115.5, 115.2, 115.0, 114.9, 114.9, 114.8, 109.7, 70.1, 64.6, 64.2, 61.7, 58.1, 47.8, 37.4, 24.7, 0.2. **MS (ESI +):** m/z (%) 687.5 ([M+H]⁺, 44).

### EXAMPLE 5: Preparation of (3R,4S)-4-(4-benzyloxyphenyl)-1-(4-fluoro phenyl)-3-{2-[2-(4-fluorophenyl)-[1,3]-dioxolan-2-yl]ethyl}azetidin-2-one (Compound VIIa)

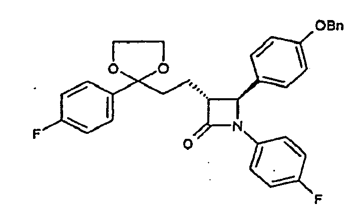

To a suspension of (*S*)-3-{(*R*)-2-[(*S*)-(4-(benzyloxyphenyl)-(4-fluorophenylamino)methyl]-4-[2-(4-fluorophenyl)-[1,3]-dioxolan-2-yl]butyryl]-4-phenyloxazolidin-2-one (4.0 g, 86% purity, 4.9 mmol) in toluene (10 mL), bis(trimethylsilyl) acetamide (BSA) (2.0 mL, 8.16 mmol) was added. The reaction mixture was stirred for 30 minutes at 50° C, and then tetrabutylammonium fluoride monohydrate (TBAF) (279 mg, 1.0 mmol) was added. After 3 hours, the reaction mixture was cooled to room temperature, and methanol (2.5 mL) was added. The reaction mixture was washed with HCl 1M (2 x 25 mL), saturated aqueous NaHCO₃ (25 mL) and brine (25 mL). The organic extracts were dried over MgSO₄, and the solvent was evaporated under vacuum. The crude product was then purified by flash chromatography (Hexane: EtOAc, 7:3) to yield (3*R*,4*S*)-4-(4-benzyloxyphenyl)-1(4-fluorophenyl)-3-{-[2-(4-fluorophenyl)-[1,3]-dioxolan-2-yl] ethyl}azetidin-2-one as an oil (2.4 g; Yield: 90%; 4.4 mmol).

*Analysis:* **¹H-NNM (400 MHz, CDCl₃) (**δ**, ppm)**: 7.50-7.20 (m, 12H), 7.08-6.92 (m, 5H), 5.07 (s, 2H), 4.61 (d, *J*= 2.3 Hz, 1H), 4.06-3.99 (m, 2H), 3.82-3.75 (m, 2H), 2.19-1.89 (m, 4H); **¹³C-NMR (125 MHz, CDCl₃) (**δ**, ppm)**: 167.2, 163.5, 161.1, 158.7, 137.8, 129.6, 129.1, 127.3, 125.9, 118.4, 118.3, 115.6, 115.4, 115.3, 114.7, 109.3, 69.8, 64.4, 60.7, 60.1, 37.5, 22.8; **MS (ESI +)**: m/z (%) = 564.1 ([M+Na] ⁺, 100), 542.2 ([M+H] ⁺, 15).

### EXAMPLE 6: Preparation of (3R,4S)-4-(4-benzyloxyphenyl)-1-(4-fluoro phenyl)-3-{2-[2-(4-fluorophenyl)-[1,3]-dioxolan-2-yl]ethyl}azetidin-2-one (Compound VIIa)

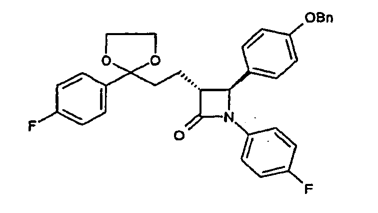

To a suspension of (*S*)-3-{(*R*)-2-[(*S*)-(4-(benzyloxyphenyl)-(4-fluorophenylamino)methyl]-4-[2-(4-fluorophenyl)-[1,3]-dioxolan-2-yl]butyryl}-4-phenyloxazolidin-2-one (10.0 g, 14.19 mmol) in toluene (95.5 mL), *N,O-*bis(trimethylsilyl)acetamide (BSA) (5.8 mL, 23.55 mmol) was added. The reaction mixture was stirred for 1 hour at 50° C, and then tetrabutylammonium fluoride hydrate (TBAF) (0.74 g, 2.84 mmol) was added. After 5 hours, the reaction mixture was cooled to room temperature, and methanol (10 mL) was added. The reaction mixture was washed with HCl 1M (2 x 40 mL), saturated aqueous sodium hydrogencarbonate (2 x 40 mL) and water (40 ml). The organic phase was concentrated under vacuum. Then ethanol (40 mL) was added, and the solvent was evaporated under vacuum. The residue was treated with ethanol (70 mL) under stirring at room temperature, precipitating a white solid that after filtering yielded (3*R*,4*S*)-4-(4-benzyloxyphenyl)-1-(4-fluorophenyl)-3-{2-[2-(4-fluorophenyl)-[1,3]-dioxolan-2-yl] ethyl}azetidin-2-one (7.3 g; Yield: 95%; 13.5 mmol).

### EXAMPLE 7: Preparation of (3R,4S)-1-(4-fluorophenyl)-3-{2-[2-(4-fluorophenyl)-1,3-dioxolan-2-yl]-ethyl}-4-(4-hydroxyphenyl)-azetidin-2-one (Compound VIIb)

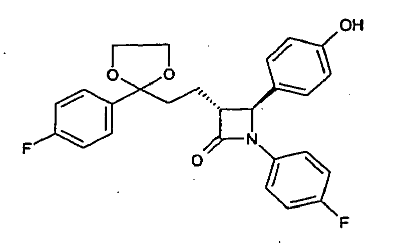

In a 100 mL flask, 4.0 g (5.8 mmol) of (*S*)-3-{(*R*)-2-[(*S*)-(4-fluorophenylamino)-(4-trimethylsilyloxyphenyl)methyl)-4-[2-(4-fluorophenyl)-[1,3]-dioxolan-2-yl]butyryl }-4-phenyloxazolidin-2-one were suspended in toluene (40 mL). Then *N*,*O-*bis(trimethylsilyl)acetamide (2.8 mL, 11.6 mmol) and the reaction mixture were heated to 50° C. Next, tetrabutylammonium fluoride hydrate (76 mg, 0.29 mmol) was added, and the reaction mixture was stirred for 5 hours at 50° C. The reaction was quenched by adding methanol (4 mL) to the reaction mixture. The organic phase was washed with HCl 0.01N (2x 20 mL), sodium hydrogencarbonate 4% (1x 20 mL) and water (20 mL), and then the solvent was evaporated under vacuum. The residue was treated with ethanol (25 mL) under stirring at room temperature, precipitating a white solid that after filtering yields (3*R*,4*S*)-1-(4-fluorophenyl)-3-{2-[2-(4-fluorophenyl)-1,3-dioxolan-2-yl]ethyl}-4-(4-trimethylsilyloxyphenyl)-azetidin-2-one (1.9 g, 0.42 mmol) (72% yield).

*Analysis:* **¹H-NMR (400 MHz, CDCl₃) (**δ**, ppm):** 7.42-7.37 (m, 2H), 7.23-7.15 (m, 4H), 7.00 (t, *J*=8.8 Hz, 2H), 6.91 (t, *J*=8.4 Hz, 2H), 6.83-6.79 (m, 2H), 5.13 (s, 1H), 4.53 (d, *J*= 2.4 Hz, 1H), 4.06-3.96 (m, 2H), 3.80-3.71 (m, 2H), 3.05-3.00 (m, 1H), 2.12-1.80 (m, 4H); **¹³C-NMR (125 MHz, CDCl₃) (**δ**, ppm)**: 167.1, 163.0, 160.6, 159.2, 157.4, 156.8, 138.4, 133.9, 127.8, 127.6, 127.5, 118.3,118.2,115.9 115.7 115.6, 115.0, 114.8, 108.9, 64.3, 59.6, 59.2, 56.0, 36.9, 22.6, 18.5; **MS (ESI +)**: m/z (%) 452.4 ([M+H]⁺, 100).

### EXAMPLE 8: Preparation of (3R,4S)-4-(4-(benzyloxy)phenyl)-1-(4-fluoro phenyl)-3-[3-(4-fluorophenyl)-3-oxopropyl]azetidin-2-one (Compound IIa)

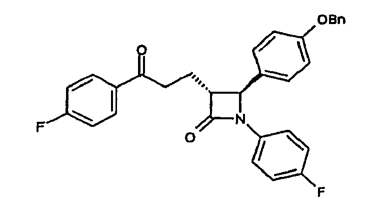

To a solution of (3*R*,4*S*)-4-(4-benzyloxyphenyl)-1-(4-fluorophenyl)-3-{2-[2-(4-fluorophenyl)-[1,3]-dioxolan-2-yl]ethyl}azetidin-2-one (2.30 g, 4.2 mmol) in acetone (25 mL) at room temperature is added *p-*toluensulfonic acid monohydrate (70 mg, 0.37 mmol). The mixture was then stirred for 6 hours at reflux temperature. The solvent was then removed under vacuum, and ethyl acetate was added (100 mL). The organic layer was then washed with water (2 x 50 mL) and dried over MgSO₄. Next, the solvent was removed under vacuum, and the crude product was purified by flash chromatography (Hexane:EtOAc, 90:10) to yield (3*R*,4*S*)-4-(4-(benzyloxy)phenyl)-1-(4-fluorophenyl)-3-[3-(4-fluorophenyl)-3-oxopropyl]-azetidin-2-one as an oil (1.71 g; Yield: 81 %; 3.4 mmol), which solidified upon standing.

*Analysis:* **¹H-NMR (400 MHz, CDCl₃) (**δ**, ppm)**: 8.01 (dd, *J*= 8.8, 5.4 Hz, 2H), 7.46-7.35 (m, 5H), 7.30-7.25 (m, 4H), 7.14 (t, *J*= 8.5 Hz, 2H), 7.00-6.93 (m, 4H), 5.07 (s, 2H), 4.70 (d, *J* = 2.3 Hz, 1H, H₄), 3.35-3.27 (m, 1H), 3.22-3.13 (m, 2H), 2.47-2.38 (m, 1H), 2.34-2.25 (m, 1H); **¹³C-NMR (125 MHz, CDCl₃) (**δ**, ppm):** 197.4,167.2,159.1, 157.7, 136.6, 133.8, 130.7, 130.6, 129.5, 128.6, 128.1, 127.4, 127.2, 118.5, 118.4, 115.9, 115.8, 115.6, 70.1, 61.1, 59.8, 35.5, 23.2; **MS (ESI+)**: m/z (%) = 536.2 ([M+K]⁺, 5), 520.2 ([M+Na]⁺, 100), 498.2 ([M+H]⁺, 5).

### EXAMPLE 9: Preparation of (3R,4S)-4-(4-(benzyloxy)phenyl)-1-(4-fluoro phenyl)-3-[3-(4-fluorophenyl)-3-oxopropyl]azetidin-2-one (Compound IIa)

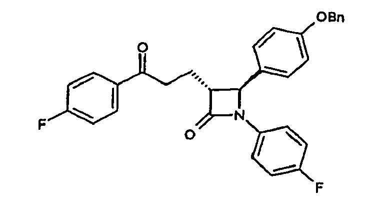

To a solution of (3*R*,4*S*)-4-(4-benzyloxyphenyl)-1-(4-fluorophenyl)-3-{2-[2-(4-fluorophenyl)-[1,3]-dioxolan-2-yl]ethyl}azetidin-2-one (7-7 g, 14.2 mmol) in wet acetone (64.7 mL acetone and 4.2 mL of water) at room temperature was added *p-*toluensulfonic acid monohydrate (0.27 g, 1.42 mmol). The mixture was stirred for 6 hours at reflux temperature. The solvent was then evaporated under vacuum, and ethyl acetate was added (80 mL). The organic layer was washed with 5% aqueous sodium hydrogencarbonate solution (2 x 80 mL) and with water (2 x 80 mL) and dried over sodium sulfate. The solvent was evaporated under vacuum. (3*R*,4*S*)-4-(4-(benzyloxy)phenyl)-1-(4-fluorophenyl)-3-[3-(4-fluorophenyl)-3-oxopropyl]-azetidin-2-one was obtained as an oily product (7.24 g; 99% yield; 97% purity; 14.1 mmol).

### EXAMPLE 10: Preparation of (3R,4S)-1-(4-fluorophenyl)-3-[3-(4-fluorophenyl)-3-oxopropyl]-4-(4-hydroxyphenyl)-azetidin-2-one (Compound IIb)

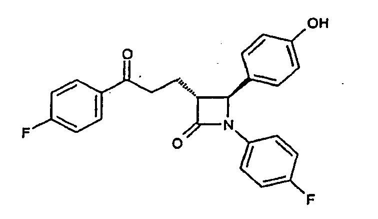

In a 50 mL flask, (3*R*,4*S*)-1-(4-fluorophenyl)-3-{2-[2-(4-fluorophenyl)-1,3-dioxolan-2-yl]ethyl}-4-(4-hydroxyphenyl)-azetidin-2-one (60 mg, 0.133 mmol) was suspended in a mixture of acetone (3 mL) and water (0.15 mL). To the resulting solution, *p*-toluensulfonic acid monohydrate (10 mg, 0.053 mmol) was added, and the reaction mixture was brought to reflux temperature for 6 hours. Then, the solvent was evaporated under vacuum, and the residue dissolved in toluene (7 mL). The organic phase was washed with 5% aqueous sodium hydrogencarbonate solution (5 mL) and with water (5 mL) and dried over sodium sulfate. The solvent was evaporated under vacuum to yield (3*R*,4*S*)-1-(4-fluorophenyl)-3-[3-(4-fluorophenyl)-3-oxopropyl]-4-(4-hydroxyphenyl)-azetidin-2-one (49 mg, 0.12 mmol) (91 % yield).
*Analysis:* **¹H-NMR (400 MHz, CDCl₃) (**δ**, ppm)**: 8.00-7.96 (m, 2H), 7.29-7.08 (m, 6H), 6.93 (t, *J* = 8.4 Hz, 2H), 6.84-6.81 (m, 2H), 5.54 (s, 1H), 4.66 (d, *J* = 2.0 Hz, 1H), 3.33-3.24 (m, 1H), 3.20-3.12 (m, 2H), 2.43-2.36 (m, 1H), 2.31-2.22 (m, 1H); **MS (ESI +):** m/z (%) 408.4 ([M+H]⁺, 100), 430.3 ([M+Na]⁺, 67), 446.2 ([M+K]⁺, 11).

### EXAMPLE 11: Preparation of (3R,4S)-4-(4-(benzyloxy)phenyl)-1-(4-fluoro phenyl)-3-[(3S)-3-(4-fluorophenyl)-3-hydroxypropyl]azetidin-2-one and (3R,4S)-1-(4-fluorophenyl)-3-[(3,S)-3-(4-fluorophenyl)-3-hydroxypropyl]-4-(4-hydroxyphenyl)azetidin-2-one (ezetimibe)

### Borane reduction

In an inert 100 mL flask, (3*R*,4*S*)-4-(4-(benzyloxy)phenyl)-1-(4-fluorophenyl)-3-[3-(4-fluorophenyl)-3-oxopropyl]azetidin-2-one (5.0 g, 10.0 mmol) were dissolved in anhydrous tetrahydrofuran (50 mL). The solution was heated to 40° C, and (*R*)-2-methyl-CBS-oxazaborolidine solution 1M in toluene (1 mL ,1.0 mmol) was charged. Then, borane diethylaniline complex (1.34 mL, 7.5 mmol) were added in one hour. After checking the reaction completion by TLC, it was quenched with methanol (15 mL). The solvent was evaporated, and the residue was redissolved in ethyl acetate (60 mL), washed with hydrogen chloride 5% (35 mL), sodium hydrogencarbonate 5% (35 mL) and water (40 mL). The solvent was evaporated, and the residue was crystallized from methanol to give (3*R*,4*S*)-4-(4-(benzyloxy)phenyl)-1-(4-fluorophenyl)-3-[(3*S*)-3-(4-fluorophenyl)-3-hydroxypropyl]azetidin-2-one (3.27 g, 6.5 mmol, 65% yield).

### Debenzylation

In an inert 100 mL flask, (3*R*,4*S*)-4-(4-(benzyloxy)phenyl)-1-(4-fluorophenyl)-3-[(3*S*)-3-(4-fluorophenyl)-3-hydroxypropyl]azetidin-2-one (0.44 g, 0.88 mmol) and Pd/C (0.1 g) were suspended in ethanol (7.1 mL). The reaction took place under hydrogen atmosphere at room temperature and was followed by TLC. Afterwards, the suspension was filtered over celite and the solvent was eliminated. The residue was crystallized in MeOH/H₂0 to give (3*R*,4*S*)-1-(4-fluorophenyl)-3-[(3*S*)-3-(4-fluorophenyl)-3-hydroxypropyl]-4-(4-hydroxyphenyl)azetidin-2-one (0.20 g, 0.488 mmol, yield 56%).

### EXAMPLE 12: Preparation of (3R,4S)-1-(4-fluorophenyl)-3-[(3S)-3-(4-fluorophenyl)-3-hydroxypropyl]-4-(4-hydroxyphenyl)azetidin-2-one (ezetimibe)

In an inert 50 mL flask, (3*R*,4*S*)-1-(4-fluorophenyl)-3-[3-(4-fluorophenyl)-3-oxopropyl]- 4-(4-hydroxyphenyl)-azetidin-2-one (0.25 g, 0.6 mmol) was dissolved in anhydrous tetrahydrofuran (3 mL). The solution was heated to 40° C, and (*R*)-2-methyl-CBS-oxazaborolidine solution 1 M in toluene (0.1 mL, 0.1 mmol) was charged. Then, borane diethylaniline complex (0.1 ml, 0.6 mmol) were added in one hour. After checking the reaction completion by TLC, it was quenched with methanol (0.5 mL). The solvent was evaporated, and the residue was redissolved in ethyl acetate (10 mL), washed with hydrogen chloride 5% (7 mL), sodium hydrogencarbonate 5% (7 mL) and water (10 mL). The solvent was evaporated, and the residue was crystallized from methanol to give (3*R*,4*S*)-1-(4-fluorophenyl)-3-[(3*S*)-3-(4-fluorophenyl)-3-hydroxypropyl]-4-(4-hydroxyphenyl)azetidin-2-one (0.2024 g , 0.494 mmol, 80.6%).

## Claims

1. A process for preparing a compound of Formula II 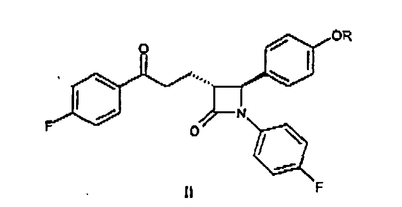 wherein R is a hydrogen, or hydroxyl protecting group comprising
i. reacting a ketone of Formula III 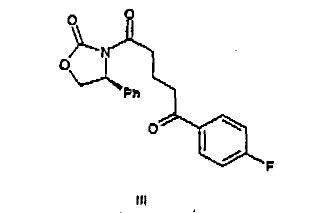 with a diol to obtain a ketal of Formula IV 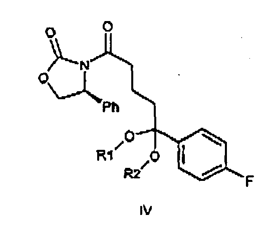 wherein R1 and R2 are together an ethylene diradical or a trimethylene diradical that may optionally be substituted with a C₁₋₄-alkyl chain;
ii. condensing said ketal of Formula IV with an imine of Formula V 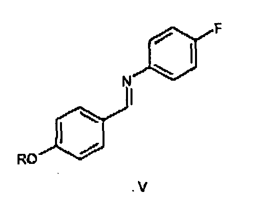 to obtain an amide of Formula VI 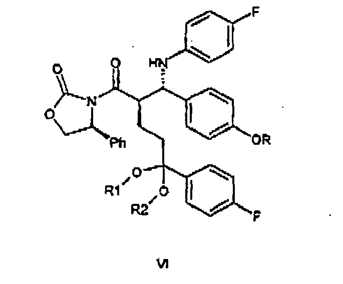
iii. cyclizing said amide of Formula VI to obtain a lactam of Formula VII 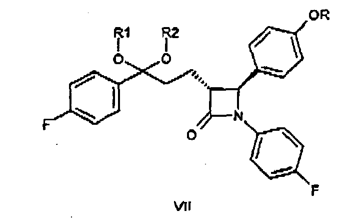 and
iv. cleaving the ketal function of said lactam of Formula VII to obtain said compound of Formula II.

2. The process of claim 1, wherein the hydroxyl protecting group is a benzyl or a trimethylsilyl group.

3. The process of claim 1, wherein R1 and R2 are together an ethylene diradical.

4. A compound of Formula IV, wherein R1 and R2 are together an ethylene diradical.

5. A compound of Formula VI, wherein R1 and R2 are together an ethylene diradical and R is a hydrogen or a hydroxyl protecting group.

6. A compound according to claim 5, wherein the compound is (*S*)-3-{(*R*)-2-[(*S*)-(4-(benzyloxyphenyl))-(4-fluorophenylamino)methyl]-4-[2-(4-fluorophenyl)-[1,3]-dioxolan-2-yl]butyryl}-4-phenyloxazolidin-2-one).

7. A compound according to claim 5, wherein the compound is (*S*)-3-{(*R*)-2-[(*S*)-(4-fluorophenylamino)-(4-hydroxyphenyl)methyl]-4-[2-(4-fluorophenyl)-[1,3]-dioxolan-2-yl]butyryl}-4-phenyloxazolidin-2-one).

8. A compound according to claim 5, wherein the compound is (*S*)-3-{(*R*)-2-[(*S*)-(4-fluorophenylamino)-(4-trimethylsilyloxyphenyl)methyl]4-[2-(4-fluorophenyl)-[1,3]-dioxolan-2-yl]butyryl}-4-phenyloxazolidin-2-one).

9. A compound of Formula VII, wherein R1 and R2 are together an ethylene diradical and R is a hydrogen or a hydroxyl protecting group.

10. A compound according to claim 9, wherein the compound is (3*R*,4*S*)-4-(4-(benzyloxyphenyl)-1-(4-fluorophenyl)-3-{2-[2-(4-fluorophenyl)-[1,3]-dioxolan-2-yl]ethyl}azetidin-2-one).

11. A compound according to claim 9, wherein the compound is (3*R*,4*S*)-1-(4-fluorophenyl)-3-{2-[2-(4-fluorophenyl)-1,3-dioxolan-2-yl]ethyl}-4-(4- trimethylsilyloxyphenyl)-azetidin-2-one).

12. A compound according to claim 9, wherein the compound is (3*R*,4*S*)-1-(4-fluorophenyl)-3-{2-[2-(4-fluorophenyl)-1,3-dioxolan-2-yl]ethyl}-4-(4-hydroxyphenyl)-azetidin-2-one).

13. The process of claim 1, further comprising the steps of:
i. performing a chiral reduction of the compound of Formula II, and
ii. removing the hydroxyl protecting group if necessary, to obtain ezetimibe.

14. The process of claim 13, wherein said chiral reduction comprises performing a borane catalyzed reduction.

15. The process of claim 13, wherein said compound of Formula II is (3*R*,4*S*)-4-(4-(benzyloxy)phenyl)-1-(4-fluorophenyl)-3-[3-(4-fluorophenyl)-3-oxopropyl] azetidin-2-one, (3*R*,4*S*)-1-(4-fluorophenyl)-3-[3-(4-fluorophenyl)-3-oxopropyl]-4-(4-(hydroxyphenyl) azetidin-2-one or (3*R*,4*S*)-4-(4-(trimethylsilyloxy)phenyl)-1-(4-fluorophenyl)-3-[3-(4-fluorophenyl)-3-oxopropyl] azetidin-2-one.

## Patentansprüche

1. Verfahren zum Herstellen einer Verbindung der Formel II 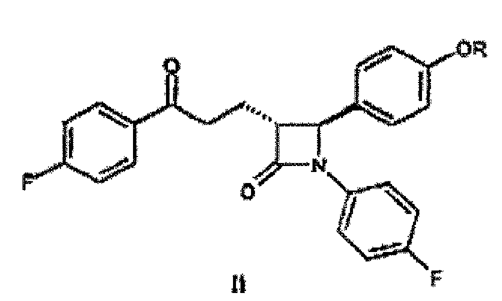 wobei R ein Wasserstoff oder eine Hydroxylschutzgruppe ist, wobei das Verfahren folgendes umfaßt:
i. Umsetzer eines Ketons der Formel III 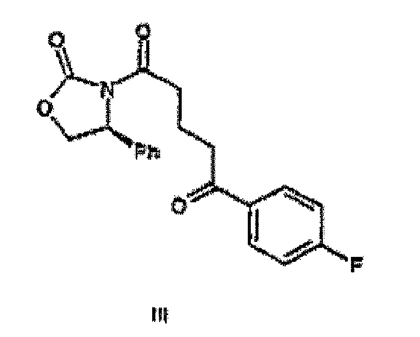 mit einem Diol ureter Erhalt eines Ketals der Formel IV 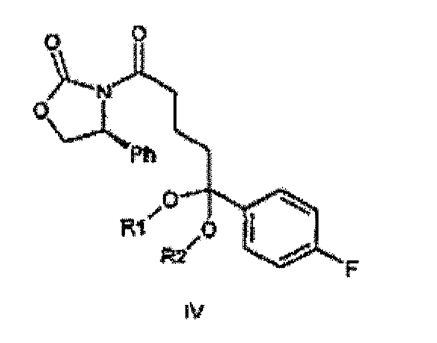 wobei R1 und R2 zusammen ein Ethylendiradikal oder ein Trimethylendiradikal sind, das optional mit einer C₁₋₄-Alkylkette substituiert sein kann,
ii. Kondensieren des Ketals der Formel IV mit einem Imin der Formel V 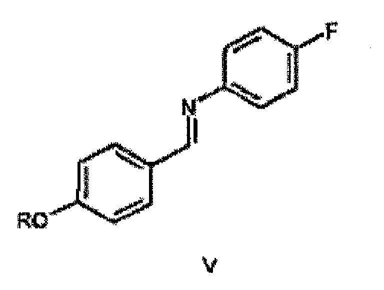 unter Erhalt eines Amids der Formen VI 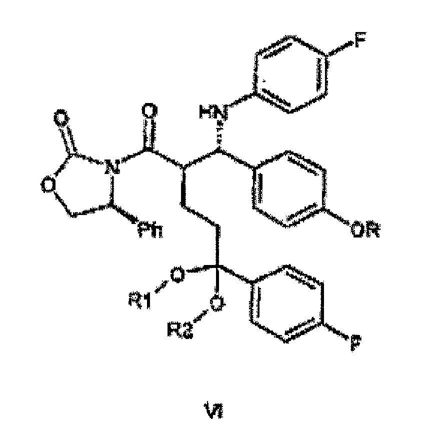
iii. Zyklisieren des Amids der Formel VI unter Erhalt eines Lactams der Formel VII 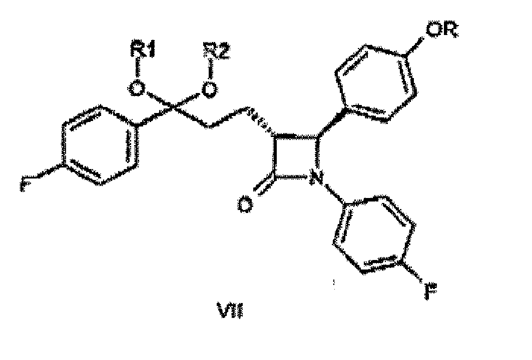 und
iv. Abspalten der Ketalfunktion von dem Lactam der Formel VII unter Erhalt der Verbindung der Formel II.

2. Verfahren nach Anspruch 1, wobei die Hydroxylschutzgruppe eine Benzyl- oder eine Trimethylsilylgruppe ist.

3. Verfahren nach Anspruch 1, wobei R1 und R2 zusammen ein Ethylendiradikal sind.

4. Verbindung der Formel IV, wobei R1 und R2 zusammen ein Ethylendiradikal sind.

5. Verbindung der Formel VI, wobei R1 und R2 zusammen ein Ethylendiradikal sind und R ein Wasserstoff oder eine Hydroxylschutzgruppe ist.

6. Verfahren nach Anspruch 5, wobei die Verbindung (*S*)-3-{(*R*)-2-[(*S*)-(4-(Benzyloxyphenyl))-(4-fluorphenylamin)-methyl]-4-[2-(4-fluorphenyl)-[1,3]-dioxolan-2-yl]-butyryl}-4-phenyloxazolidin-2-on) ist.

7. Verbindung nach Anspruch 5, wobei die Verbindung (*S*)-3-{(*R*)-2-[(*S*)-(4-Fluorphenylamin)-(4-hydroxyphenyl)-methyl]-4-[2-(4-fluorphenyl)-[1,3]-dioxolan-2-yl]-butyryl)-4-phenyloxazolidin-2-on) ist.

8. Verbindung nach Anspruch 5, wobei die Verbindung (*S*)-3-{(*R*)-2-[(*S*)-(4-Fluorphenylamin)-(4-trimethylsilyloxyphenyl)-methyl]-4-[2-(4-fluorphenyl)-[1,3]-dioxolan-2-yl]-butyryl}-4-phenyl-oxazolidin-2-on) ist.

9. Verbindung der Formel VII, wobei R1 und R2 zusammen ein Ethylendiradikal sind und R Wasserstoff oder eine Hydroxylschutzgruppe ist.

10. Verbindung nach Anspruch 9, wobei die Verbindung (3*R*,4*S*)-4-(4-Benzyloxyphenyl)-1-(4-fluorphenyl}-3-{2-[2-(4-fluorphenyl)-[1,3]-dioxolan-2-yl]-ethyl}-azetidin-2-on) ist.

11. Verbindung nach Anspruch 9, wobei die Verbindung (3*R*,4*S*)-1-(4-Fluorphenyl)-3-{2-[2-(4-fluorpohenyl)-1,3-dioxolan-2-yl]-ethyl}-4-{4-trimethylsilyloxyphenyl}-azetidin-2-on} ist.

12. Verbindung nach Anspruch 9, wobei die Verbindung (3*R*,4*S*)-1-(4-Fluorphenyl)-3-{2-[2-(4-fluorphenyl)-1,3-dioxolan-2-yl]-ethyl}-4-(4-hydroxyphenyl)-azetidin-2-on) ist.

13. Vorfahren nach Anspruch 1, welches weiterhin die Stufen umfaßt, bei denen man:
i. eine chirale Reduktion der Verbindung der Formel II durchführt und
ii. die Hydroxylschutzgruppe notwendigenfalls entfernt,
um Ezetimibe zu erhalten.

14. Verfahren nach Anspruch 13, wobei die chirale Reduktion das Durchführen einer durch Boran katalysierten Reduktion umfaßt.

15. Verfahren nach Anspruch 13, wobei die Verbindung der Formel II (3*R*,4*S*)-4-(4-Benzyloxy)-phenyl)-1-(4-fluorphenyl)-3-[3-(4-fluorphenyl)-3-oxopropyl]-azetidin-2-on, (3*R*,4*S*)-1-(4-Fluorphenyl)-3-[3-(4-fluorphenyl)-3-oxopropyl]-4-(4-(hydroxyphenyl)-azetidin-2-on oder (3*R*,4*S*)-4-(4-(Trimethylsilyloxy)-phenyl-1-(4-fluorphenyl)-3-[3-(4-fluorphenyl)-3-oxopropyl]-azetidin-2-on ist.

## Revendications

1. Procédé de préparation d'un composé de formule II 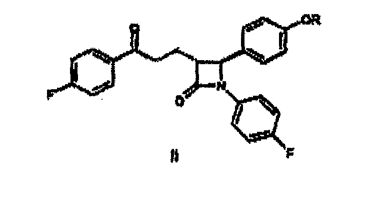 dans lequel R est un hydrogène, ou un groupe de protection d'un hydroxyle comprenant :
i. la réaction d'une cétone de formule III 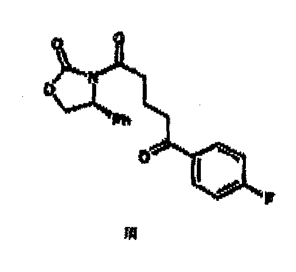 avec un diol pour obtenir un cétal de formule IV 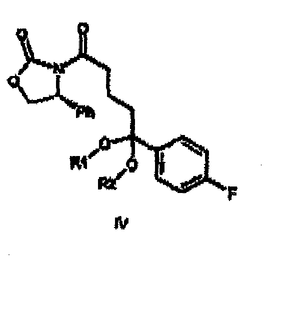 dans lequel R1 et R2 sont ensemble un diradical éthylène ou un diradical triméthylène qui peuvent éventuellement être substitués par une chaîne alkyle en C₁₋₄ ;
ii. la condensation dudit cétal de formule IV avec une imine de formule V 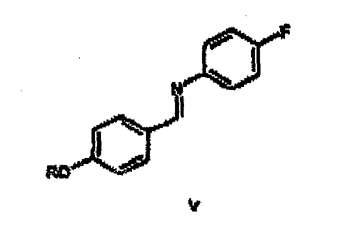 pour obtenir un amide de formule VI 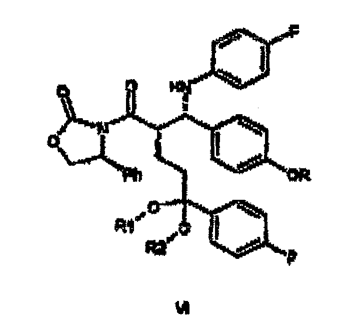
iii. la cyclisation dudit amide de formule VI afin d'obtenir un lactame de formule VII 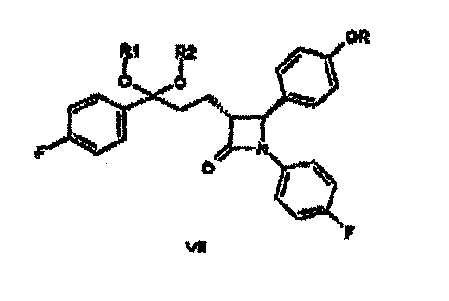 et
iv. le clivage de la fonction cétal dudit lactame de formule VII pour obtenir ledit composé de formule II.

2. Procédé selon la revendication 1, dans lequel le groupe de protection d'un hydroxyle est un groupe benzyle ou triméthylsilyle.

3. Procédé selon la revendication 1, dans lequel R1 et R2 sont ensemble un diradical éthylène.

4. Composé de formule IV, dans lequel R1 et R2 sont ensemble un diradical éthylène.

5. Composé de formule VI, dans lequel R1 et R2 sont ensemble un diradical éthylène et R est un hydrogène ou un groupe de protection d'un hydroxyle.

6. Composé selon la revendication 5, dans lequel le composé est une (*S*)-3-{(*R*)-2-[(*S*)-(4-(benzyloxyphényl)-(4-fluorophénylamino)méthyl]-4-[2-(4-fluorophényl)-[1,3]-dioxolan-2-yl]butyryl}-4-phényloxazolidin-2-one).

7. Composé selon la revendication 5, dans lequel le composé est une (*S*)-3-{(*R*)-2-[(*S*)-(4-fluorophénylamino)-(4-hydroxyphényl)méthyl]-4-[2-(4-fluorophényl)-[1,3]-dioxolan-2-yl]butyryl}-4-phényloxazolidin-2-one).

8. Composé selon la revendication 5, dans lequel le composé est une (*S*)-3-{(*R*)-2-[(*S*)-(4-fluorophénylamino)-(4-triméthylsilyloxyphényl)méthyl]-4-[2-(4-fluoraphényl)-[1,3]-diaxolan-2-yl]butyryl}-4-phényloxazolidin-2-one).

9. Composé de formule VII, dans lequel R1 et R2 sont ensemble un diradical éthylène et R est un hydrogène ou un groupe de protection d'un hydroxyle.

10. Composé selon la revendication 9, dans lequel le composé est une (3*R*-4*S*)-4-(4-(benzyloxyphényl)-1-(4-fluorophényl)-3-{2-[2-(4-fluorophényl)-[1,3]-dioxolan-2-yl]éthyl}-azétidin-2-one).

11. Composé selon la revendication 9, dans lequel le composé est une (3*R*-4*S*)-1-(4-fluorophényl)-3-{2-[2-(4-fluorophényl)-1,3-dioxalan-2-yl]éthyl}-4-(4-triméthylsilyloxyphényl)-azétidin-2-one).

12. Composé selon la revendication 9, dans lequel le composé est une (3*R*-4*S*)-1-(4-fluorophényl)-3-{2-[2-(4-fluorophényl)-1,3-dioxolan-2-yl]éthyl}-4-(4-hydroxyphényl)-azétidin-2-one).

13. Procédé selon la revendication 1, comprenant en outre les étapes consistant à :
i. effectuer une réduction chirale du composé de formule II, et
ii. enlever le groupe de protection d'un hydroxyle si nécessaire,
pour obtenir l'ezetimibe.

14. Procédé selon la revendication 13, dans lequel ladite réduction chirale comprend la réalisation d'une réduction catalysée du borane.

15. Procédé selon la revendication 13, dans lequel ledit composé de formule Il est une (3*R*-4*S*)-4-(4-(benzyloxy)phényl)-1-(4-fluorophényl)-3-[3-(4-fluorophényl)-3-oxopropyl]-azétidin-2-one, une (3*R*-4*S*)-1-(4-fluorophényl)-3-[3-(4-fluorophényl)-3-oxopropyl]-4-(4-hydroxyphényl)-azétidin-2-one ou une (3*R*-4*S*)-4-(4-(triméthylsilyloxy)phényl)-1-(4-fluorophényl)-3-[3-(4-fluorophényl)-3-oxopropyl]-azétidin-2-one.
